(19) Europäisches Patentamt / European Patent Office / Office européen des brevets

(11) **EP 2 365 455 A1**

(12) **DEMANDE DE BREVET EUROPEEN**

(43) Date de publication:
**14.09.2011 Bulletin 2011/37**

(51) Int Cl.:
*G06F 19/00* (2011.01)　　　*A61B 5/0275* (2006.01)
*G06K 9/46* (2006.01)　　　*G06T 7/00* (2006.01)

(21) Numéro de dépôt: **11305247.6**

(22) Date de dépôt: **08.03.2011**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **10.03.2010 FR 1051738**

(71) Demandeur: **Commissariat à l'Énergie Atomique et aux Énergies Alternatives 75015 Paris (FR)**

(72) Inventeurs:
• **Maroy, Renaud**
**75015, PARIS (FR)**
• **De Gavriloff, Ségolène**
**91120, Palaiseau (FR)**

(74) Mandataire: **Jacobson, Claude**
**Cabinet Lavoix**
**2, Place d'Estienne d'Orves**
**75441 Paris Cedex 09 (FR)**

(54) **Procédé d'extraction simultanée de la fonction d'entrée et des paramètres pharmacocinétiques d'un principe actif**

(57)　Ce procédé d'extraction dans une structure d'un organisme vivant à partir d'une image de mesure de la cinétique du principe actif comprenant une pluralité de régions d'intérêt à l'intérieur de chacune desquelles la cinétique du principe actif est mesurée, comprend les étapes suivantes :
- détermination (30) d'une pluralité de jeux de cinétiques, les jeux de cinétiques déterminés étant différents les uns des autres et chaque jeu de cinétiques déterminé comportant plusieurs cinétiques mesurées ;

- définition (32) d'au moins un modèle de cinétique, des paramètres du modèle de cinétique défini étant formés par les paramètres de la fonction d'entrée et les paramètres pharmacocinétiques ;
- estimation (34) de la fonction d'entrée et des paramètres pharmacocinétiques par résolution du modèle de cinétique défini à partir du jeu de cinétiques déterminé ; et
- déduction (36) de la fonction d'entrée finale et des paramètres pharmacocinétiques finaux.

EP 2 365 455 A1

**Description**

La présente invention concerne un procédé d'extraction simultanée de la fonction d'entrée et des paramètres pharmacocinétiques d'un principe actif dans une structure d'un organisme vivant à partir d'une image de mesure de la cinétique du principe actif dans la structure obtenue par un appareil d'imagerie, l'image comprenant une pluralité de régions d'intérêt à l'intérieur de chacune desquelles la cinétique du principe actif est mesurée.

L'imagerie moléculaire in vivo permet de mesurer des paramètres pharmacocinétiques d'un principe actif dans des animaux et êtres humains intacts. L'imagerie moléculaire englobe des techniques nucléaires et des techniques optiques. Parmi les techniques nucléaires, la tomographie par émission de positons (TEP) ainsi que la tomographie par émission monophotonique (TEMP) sont les techniques les plus sensibles et les seules à offrir des mesures quantitatives.

La quantification des mesures avec la TEP ou la TEMP pour l'étude d'un principe actif nécessite l'injection intraveineuse d'un traceur radioactif adapté à l'étude de ce principe actif. Une image de la distribution du traceur, et donc du principe actif, est alors reconstruite à partir d'une séquence temporelle d'images TEP ou TEMP de mesure de la concentration du traceur.

Le post-traitement de l'image TEP ou TEMP requiert en général trois étapes pour accéder aux paramètres pharmacocinétiques. Tout d'abord, des régions d'intérêt délimitant les organes pharmacocinétiques, appelés pharmaco-organes, doivent être définis sur la séquence temporelle d'images TEP ou TEMP, ou sur une image morphologique recalée avec la séquence temporelle d'images TEP ou TEMP. Ensuite, les cinétiques des pharmaco-organes, c'est-à-dire les courbes d'activité temporelle du traceur correspondant aux variations au fil du temps de la concentration du traceur, doivent être extraites. Enfin, un modèle physiologique de cinétique peut être défini, sur la base des cinétiques et de la concentration du traceur dans le plasma, permettant le calcul des paramètres pharmacocinétiques intéressants pour le praticien.

L'extraction des paramètres pharmacocinétiques en imagerie utilisant un traceur est donc l'ultime étape donnant accès à des informations sur le traitement du traceur par l'organisme, pouvant ainsi renseigner sur l'efficacité d'un médicament ou l'évolution d'une pathologie.

On connaît des procédés permettant d'estimer les paramètres pharmacocinétiques et la concentration plasmatique du traceur non métabolisé en fonction du temps, appelée fonction d'entrée, à partir des cinétiques extraites. En effet, la fonction d'entrée est nécessaire à la connaissance des paramètres pharmacocinétiques mais elle n'est pas toujours visible directement dans l'image.

Cependant, les paramètres pharmacocinétiques extraits par ces procédés souffrent d'un très fort biais lorsqu'ils ne bénéficient pas d'informations supplémentaires sous la forme de prélèvements veineux ou artériels. Par ailleurs, l'existence d'une telle restriction ne rend ces procédés applicables qu'à un nombre très réduit de traceurs.

L'invention a pour but de proposer un procédé à caractère invasif minimal permettant d'extraire directement à partir de l'image, avec le moins de biais possible, les paramètres pharmacocinétiques simultanément avec la fonction d'entrée.

A cet effet, l'invention a pour objet un procédé du type précité caractérisé en ce qu'il comprend les étapes suivantes :

- détermination d'une pluralité de jeux de cinétiques à partir des cinétiques mesurées, les jeux de cinétiques déterminés étant différents les uns des autres et chaque jeu de cinétiques déterminé comportant plusieurs cinétiques mesurées ;
- définition d'au moins un modèle de cinétique, des paramètres du modèle de cinétique défini étant formés par les paramètres de la fonction d'entrée et les paramètres pharmacocinétiques ;
- estimation, pour chaque jeu de cinétiques déterminé, de la fonction d'entrée et des paramètres pharmacocinétiques par résolution du modèle de cinétique défini à partir du jeu de cinétiques déterminé ; et
- déduction de la fonction d'entrée finale et des paramètres pharmacocinétiques finaux à partir des fonctions d'entrée estimées et des paramètres pharmacocinétiques estimés.

Le procédé selon l'invention peut comporter une ou plusieurs des caractéristiques suivantes :

- l'étape de détermination de la pluralité de jeux de cinétiques comprend les étapes suivantes :
- constitution de tous les jeux de cinétiques possibles à partir des cinétiques mesurées ; et
- sélection, parmi les jeux de cinétiques constitués, d'un nombre prédéterminé de jeux de cinétiques, cette étape comprenant les étapes suivantes :

  • évaluation, pour chaque jeu de cinétiques constitué, d'un critère de sélection de jeux de cinétiques permettant d'évaluer leur risque d'introduire des erreurs dans l'estimation de la fonction d'entrée et des paramètres pharmacocinétiques ;
  • classement des jeux de cinétiques constitués selon une règle du critère de sélection maximal ; et

- choix du nombre prédéterminé des premiers jeux de cinétiques classés ;

  - le critère de sélection de jeux de cinétiques permet de sélectionner les jeux de cinétiques dont les cinétiques mesurées sont différentes les unes des autres en termes de forme et/ou d'amplitude, et/ou dont les cinétiques mesurées sont peu affectées par l'effet de volume partiel et/ou dont les cinétiques mesurées présentent un fort rapport signal sur bruit ;
  - le modèle de cinétique défini prend en compte la métabolisation du principe actif dans la structure de l'organisme vivant imagé ;
  - l'étape d'estimation de la fonction d'entrée et des paramètres pharmacocinétiques est réalisée plusieurs fois pour chaque jeu de cinétiques déterminé de manière à trouver les différentes solutions du modèle de cinétique défini ;
  - les jeux de cinétiques déterminés comprennent des jeux de cinétiques comportant le même nombre de cinétiques mesurées ;
  - les jeux de cinétiques déterminés comprennent des jeux de cinétiques comportant un nombre différent de cinétiques mesurées ;
  - l'étape d'estimation de la fonction d'entrée et des paramètres pharmacocinétiques comprend la minimisation d'une fonction de coût permettant d'évaluer la dissemblance entre le jeu de cinétiques déterminé et le modèle de cinétique défini ;
  - l'étape d'estimation de la fonction d'entrée et des paramètres pharmacocinétiques est réalisée suivant une boucle itérative dans laquelle les fonctions d'entrée estimées et les paramètres pharmacocinétiques estimés lors d'une itération permettent de déterminer l'initialisation des fonctions d'entrée estimées et des paramètres pharmacocinétiques estimés à l'itération suivante et/ou d'apporter des contraintes sur les fonctions d'entrée estimées et les paramètres pharmacocinétiques estimés à l'itération suivante ;
  - l'étape de définition d'au moins un modèle de cinétique comprend la définition d'une pluralité de modèles de cinétique, des paramètres des modèles de cinétique définis étant formés par les paramètres de la fonction d'entrée et les paramètres pharmacocinétiques ;
  - l'étape d'estimation de la fonction d'entrée et des paramètres pharmacocinétiques comprend, pour chaque jeu de cinétiques déterminé, la résolution des modèles de cinétique définis à partir du jeu de cinétiques déterminé, et le procédé comprend une étape de choix, parmi la pluralité de modèles de cinétique, d'un modèle de cinétique optimal en termes de compromis entre l'adéquation du modèle de cinétique aux données et le faible nombre de paramètres de la fonction d'entrée et des paramètres pharmacocinétiques à extraire ;
  - l'étape de définition d'au moins un modèle de cinétique comprend, outre le choix de la forme du modèle de cinétique, l'introduction dans le modèle de cinétique de contraintes résultant de connaissances a priori non liées à des prélèvements artériels effectués sur l'organisme vivant imagé, les contraintes permettant de fixer au moins un paramètre du modèle de cinétique tel que les fractions vasculaires des régions d'intérêt ;
  - l'étape d'estimation de la fonction d'entrée et des paramètres pharmacocinétiques comprend l'introduction dans la résolution du modèle de cinétique de contraintes résultant de connaissances a priori non liées à des prélèvements artériels effectués sur l'organisme vivant imagé, les contraintes permettant d'ajouter des termes connus dans la résolution du modèle de cinétique tels que des termes prenant en compte des connaissances a priori sur les fractions vasculaires des régions d'intérêt et/ou sur le volume de distribution du principe actif sur une population donnée ; et
  - l'image de mesure de la cinétique du principe actif dans la structure est obtenue à partir d'une séquence temporelle d'images tridimensionnelles obtenues par tomographie par émission de positons ou par tomographie par émission monophotonique ; et
  - l'étape de déduction de la fonction d'entrée finale et des paramètres pharmacocinétiques finaux est réalisée en calculant la moyenne ou la médiane des paramètres des fonctions d'entrée estimées et des paramètres pharmacocinétiques estimés.

L'invention a également pour objet un support de stockage d'information comprenant un code pour extraire simultanément la fonction d'entrée et les paramètres pharmacocinétiques d'un principe actif dans une structure d'un organisme vivant à partir d'une image de mesure de la cinétique du principe actif dans la structure obtenue par un appareil d'imagerie, l'image comprenant une pluralité de régions d'intérêt à l'intérieur de chacune desquelles la cinétique du principe actif est mesurée, caractérisé en ce que le code comprend des instructions pour :

- déterminer une pluralité de jeux de cinétiques à partir des cinétiques mesurées, les jeux de cinétiques déterminés étant différents les uns des autres et chaque jeu de cinétiques déterminé comportant plusieurs cinétiques mesurées ;
- définir au moins un modèle de cinétique, des paramètres du modèle de cinétique défini étant formés par les paramètres de la fonction d'entrée et les paramètres pharmacocinétiques ;

- estimer, pour chaque jeu de cinétiques déterminé, la fonction d'entrée et les paramètres pharmacocinétiques par résolution du modèle de cinétique défini à partir du jeu de cinétiques déterminé ; et
- déduire la fonction d'entrée finale et les paramètres pharmacocinétiques finaux à partir des fonctions d'entrée estimées et des paramètres pharmacocinétiques estimés.

L'invention a en outre pour objet un dispositif destiné à extraire simultanément la fonction d'entrée et les paramètres pharmacocinétiques d'un principe actif dans une structure d'un organisme vivant à partir d'une image de mesure de la cinétique du principe actif dans la structure obtenue par un appareil d'imagerie, l'image comprenant une pluralité de régions d'intérêt à l'intérieur de chacune desquelles la cinétique du principe actif est mesurée, caractérisé en ce qu'il comprend :

- un appareil d'imagerie; et
- un système de traitement de données relié à l'appareil d'imagerie et comprenant :

  • des moyens pour déterminer une pluralité de jeux de cinétiques à partir des cinétiques mesurées, les jeux de cinétiques déterminés étant différents les uns des autres et chaque jeu de cinétiques déterminé comportant plusieurs cinétiques mesurées ;
  • des moyens pour définir au moins un modèle de cinétique, des paramètres du modèle de cinétique défini étant formés par les paramètres de la fonction d'entrée et les paramètres pharmacocinétiques ;
  • des moyens pour estimer, pour chaque jeu de cinétiques déterminé, la fonction d'entrée et les paramètres pharmacocinétiques par résolution du modèle de cinétique défini à partir du jeu de cinétiques déterminé ; et
  • des moyens pour déduire la fonction d'entrée finale et les paramètres pharmacocinétiques finaux à partir des fonctions d'entrée estimées et des paramètres pharmacocinétiques estimés.

L'invention sera mieux comprise à la lecture de la description qui va suivre, donnée uniquement à titre d'exemple et faite en se référant aux dessins annexés, sur lesquels :

- la Figure 1 est une vue schématique d'un dispositif mettant en oeuvre le procédé selon l'invention ;
- la Figure 2 est un organigramme représentant les étapes principales du procédé selon l'invention ;
- la Figure 3 est un organigramme représentant les étapes secondaires de la première étape principale de la Figure 2 ;
- la Figure 4 est une vue schématique d'un exemple de modélisation compartimentale de processus physiologiques d'interaction entre un traceur et une région d'intérêt ;
- la Figure 5 est un organigramme représentant des étapes supplémentaires du procédé selon l'invention ;
- les Figures 6 et 7 sont des schémas présentant les résultats obtenus avec le procédé selon l'invention sur des données simulées pour respectivement la fonction d'entrée et les paramètres pharmacocinétiques ; et
- les Figures 8 et 9 sont des schémas similaires à ceux respectivement des Figures 6 et 7 présentant les résultats obtenus sur des données expérimentales ;
- les figures 10 à 13 représentent l'évolution de l'activité dans le plasma artériel de quatre sujets au cours du temps, selon différentes méthodes dont une méthode dans un mode de mise en oeuvre de l'invention.

Le procédé selon l'invention consiste à extraire simultanément la fonction d'entrée et les paramètres pharmacocinétiques d'un principe actif à partir d'une image de mesure de la cinétique du principe actif.

Pour cela, un traceur radioactif, par exemple le FDG, correspondant au principe actif étudié est administré, par exemple par injection intraveineuse, et est véhiculé par le sang jusque dans une structure d'un organisme vivant, par exemple le cerveau d'un être humain.

Toute structure d'un organisme est considérée comme un système qui agit sur le comportement du traceur. Le devenir du traceur dans la structure étudiée est ainsi modélisé par un modèle à compartiments, comme cela sera expliqué plus en détail ultérieurement. Dans ce modèle, les différents états du traceur sont modélisés par des compartiments, par exemple un compartiment représentant l'état intact du traceur dans le plasma, un compartiment représentant l'état métabolisé du traceur dans le plasma, un compartiment représentant l'état libre du traceur dans la structure, un compartiment représentant l'état lié non spécifique du traceur dans la structure, un compartiment représentant l'état lié spécifique du traceur dans la structure, etc., dans lesquels le traceur est présent à une certaine concentration. Le modèle est également caractérisé par des vitesses de transfert du traceur entre compartiments. Le nombre de compartiments et leur nature dépendent du type de traceur.

La connaissance des concentrations du traceur dans chaque compartiment et des vitesses de transfert du traceur entre compartiments, ces vitesses de transfert formant les paramètres pharmacocinétiques du traceur, permet de remonter à des quantités physiologiques, comme par exemple l'affinité du traceur à sa cible, la consommation de glucose de la structure, etc. La mesure de la concentration radioactive du traceur n'est qu'une conséquence du traitement caché du traceur par la structure et est généralement donnée par la somme pondérée des concentrations du traceur dans les différents compartiments.

Afin de remonter des mesures de concentration radioactive du traceur aux paramètres du modèle, il est nécessaire de connaître la concentration de traceur disponible aux structures, qui est la concentration de traceur non métabolisé dans le plasma sanguin en fonction du temps et appelée fonction d'entrée.

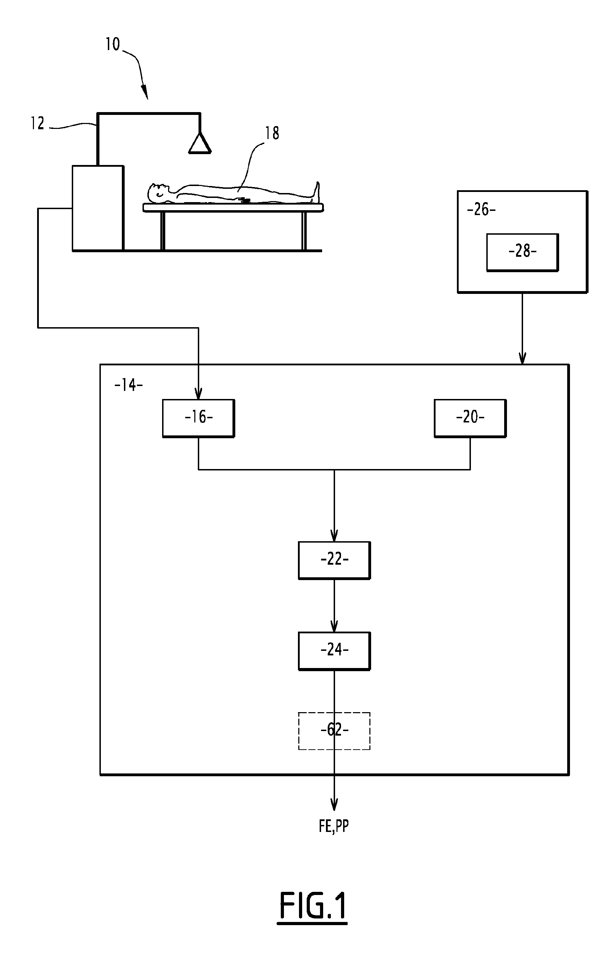

La Figure 1 représente un dispositif 10 permettant d'extraire simultanément la fonction d'entrée FE et les paramètres pharmacocinétiques PP d'un principe actif au moyen d'un traceur radioactif correspondant au principe actif étudié.

Le dispositif 10 comprend un appareil d'imagerie 12 et un système de traitement de données 14 relié à l'appareil d'imagerie 12.

Le système de traitement de données 14 comprend des moyens 16 reliés à l'appareil d'imagerie 12 pour déterminer une pluralité de jeux de cinétiques à partir de cinétiques du traceur mesurées dans une structure d'un organisme vivant 18 grâce à l'appareil d'imagerie 12, et des moyens 20 pour définir au moins un modèle de cinétique.

Un jeu de I cinétiques peut être défini comme un vecteur à I composantes dans lequel chaque composante est formée par une cinétique mesurée dans une séquence temporelle d'images de mesure au sein d'une région d'intérêt délinéant la structure considérée.

Un modèle de cinétique pour une région d'intérêt i peut être défini comme une modélisation de la cinétique du traceur dans cette région, c'est-à-dire une modélisation de la variation en fonction du temps de la concentration du traceur dans la région, certains des paramètres du modèle étant formés par les paramètres de la fonction d'entrée et les paramètres pharmacocinétiques.

Le système de traitement de données 14 comprend des moyens 22 reliés aux moyens de détermination 16 et aux moyens de définition 20 pour estimer la fonction d'entrée et les paramètres pharmacocinétiques, et des moyens 24 reliés aux moyens d'estimation 22 pour déduire la fonction d'entrée finale et les paramètres pharmacocinétiques finaux.

Les différents moyens 16, 20, 22, et 24 du système de traitement de données 14 sont mis en oeuvre à l'aide d'un support de stockage d'information 26 comprenant un code 28 comportant des instructions, chaque instruction correspondant respectivement à chacun des moyens 16, 20, 22 et 24.

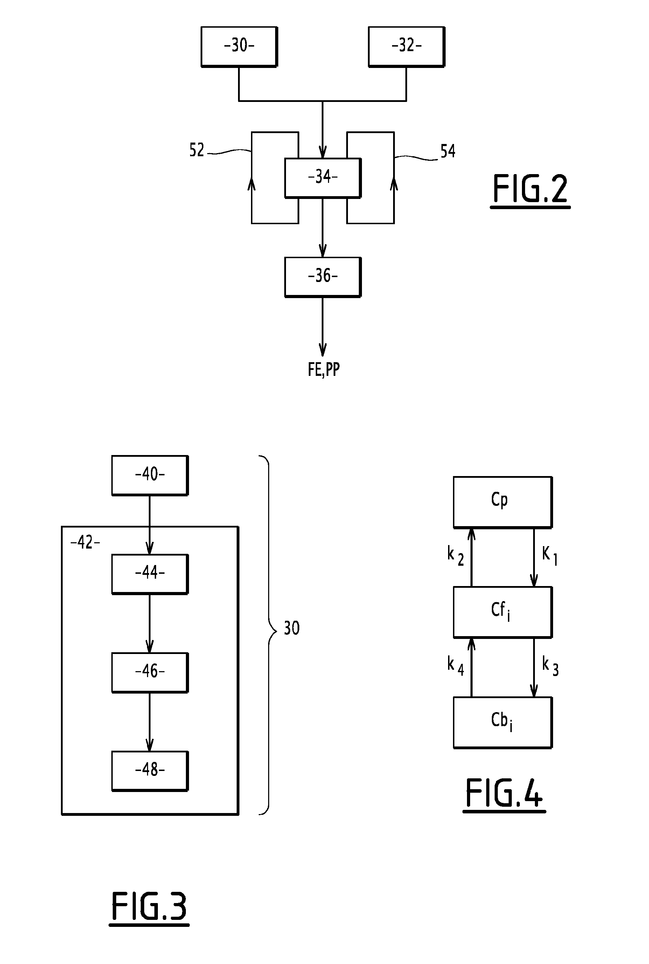

Comme illustré sur la Figure 2, le procédé selon l'invention comprend quatre étapes principales :

- une première étape 30 destinée à déterminer la pluralité de jeux de cinétiques et réalisée par les moyens de détermination 16 (Figure 1) ;
- une deuxième étape 32 destinée à définir le modèle de cinétique et réalisée par les moyens de définition 20 (Figure 1) ;
- une troisième étape 34 destinée à estimer la fonction d'entrée et les paramètres pharmacocinétiques par résolution du modèle de cinétique défini à l'étape 32 à partir des jeux de cinétiques déterminés à l'étape 30, et réalisée par les moyens d'estimation 22 (Figure 1) ; et
- une quatrième étape 36 destinée à déduire la fonction d'entrée finale et les paramètres pharmacocinétiques finaux à partir des fonctions d'entrée et des paramètres pharmacocinétiques estimés à l'étape 34, et réalisée par les moyens de déduction 24 (Figure 1).

Les quatre étapes principales 30, 32, 34 et 36 du procédé selon l'invention sont expliquées ci-après.

En référence à la Figure 3, la première étape principale 30 de détermination d'une pluralité de jeux de cinétiques comprend elle-même quatre étapes secondaires.

Initialement, un traceur radioactif correspondant au principe actif étudié est injecté au sein d'une structure d'un organisme vivant 18 et une image de mesure de la cinétique du traceur dans la structure est obtenue par l'appareil d'imagerie 12.

L'image de mesure est obtenue à partir d'une séquence temporelle d'images tridimensionnelles de mesure de la concentration du traceur dans la structure, elles-mêmes obtenues par tomographie par émission de positons (TEP) ou par tomographie par émission monophotonique (TEMP).

A l'aide d'un algorithme quelconque approprié de segmentation d'images, l'image obtenue est partitionnée en une pluralité O de régions d'intérêt.

La cinétique du traceur est alors mesurée à l'intérieur de chacune des régions d'intérêt, par exemple par un algorithme quelconque approprié de correction de l'effet de volume partiel, conduisant ainsi à O cinétiques mesurées du traceur.

Lors d'une première étape secondaire 40, tous les jeux de cinétiques possibles sont constitués à partir des O cinétiques mesurées de telle sorte que tous les jeux de cinétiques sont différents les uns des autres et que chaque jeu de cinétiques comporte plusieurs cinétiques mesurées, c'est-à-dire au moins deux cinétiques mesurées. Soit I le nombre de cinétiques par jeu, avec $2 \leq I \leq O$, alors au total $\displaystyle\sum_{l=2}^{O} C_O^l$ jeux de I cinétiques sont constitués.

Une deuxième étape secondaire 42 permet ensuite de sélectionner, parmi les jeux de cinétiques constitués, un nombre prédéterminé de jeux de cinétiques.

En effet, soit un jeu r de I cinétiques. L'estimation de la fonction d'entrée et des paramètres pharmacocinétiques sera d'autant meilleure que :

- les I cinétiques du jeu r sont différentes les unes des autres, tant d'un point de vue de la forme que de l'amplitude ;
- les I cinétiques du jeu r sont peu affectées par l'effet de volume partiel ; et
- les I cinétiques du jeu r présentent un fort rapport signal sur bruit.

Par exemple, certaines des cinétiques du jeu r peuvent être issues du même ensemble fonctionnel, ou bien de deux ensembles différents mais aux fonctions semblables et ont donc de fortes chances d'être semblables. L'étape de sélection 42 empêche ainsi de sélectionner le jeu r comportant de telles cinétiques.

L'étape de sélection 42 comprend elle-même trois sous-étapes.

Une première sous-étape 44 consiste à évaluer, pour chaque jeu de cinétiques constitué à l'étape de constitution 40, un critère $\Psi$ de sélection de jeux de cinétiques permettant d'évaluer leur risque d'introduire des erreurs dans l'estimation de la fonction d'entrée et des paramètres pharmacocinétiques :

$$\psi\left(\{\mathbf{Y_i}\}_{1\leq i \leq I}\right) = \frac{1}{I-1}\sum_{i=1}^{I-1}\left(\frac{\omega_1}{w_i^2} + \frac{1}{I-i}\sum_{j=i+1}^{I}\left(\omega_2\left(\frac{Y_i(t)}{\|Y_i\|} - \frac{Y_j(t)}{\|Y_j\|}\right)^2 + \omega_3\left(\|Y_i\| - \|Y_j\|\right)^2 + \omega_4\sum_{t=1}^{T}\frac{Y_i^2(t)}{v_{i,t}}\right)\right)$$

avec $w_i$ le coefficient de recouvrement de la région i, c'est-à-dire la proportion du signal provenant effectivement de la structure i qui contribue à la mesure moyenne de concentration d'activité dans la région i au temps t, rendant compte de l'affection de la cinétique $Y_i$ par l'effet de volume partiel, $v_{i,t}$ la variance de l'erreur commise du fait du bruit statistique dans l'image sur l'estimation de $Y_i(t)$, et $\omega_1, \omega_2, \omega_3, \omega_4$, des poids reflétant l'importance relative accordée respectivement à l'affection de la cinétique $Y_i$ par l'effet de volume partiel, à la différence entre les formes des cinétiques, à la différence entre les normes des cinétiques et au rapport signal sur bruit des cinétiques.

Une deuxième sous-étape 46 consiste ensuite à classer tous les jeux de cinétiques constitués à l'étape de constitution 40 selon une règle du critère de sélection maximal, c'est-à-dire que les jeux de cinétiques constitués sont triés par ordre de $\Psi$ décroissant.

Une troisième sous-étape 48 consiste à choisir le nombre prédéterminé de jeux de cinétiques parmi les premiers jeux de cinétiques classés à la sous-étape 46.

Lorsqu'un utilisateur choisit un nombre maximum de jeux de cinétiques à utiliser pour l'estimation de la fonction d'entrée et des paramètres pharmacocinétiques, par exemple 100, alors la sous-étape 48 consiste à choisir les 100 meilleurs jeux de cinétiques selon $\Psi$, les autres jeux de cinétiques n'étant pas utilisés pour l'estimation.

La deuxième étape principale 32 permet de définir au moins un modèle de cinétique, des paramètres du modèle de cinétique défini étant formés par les paramètres de la fonction d'entrée et les paramètres pharmacocinétiques.

Comme mentionné précédemment, les processus physiologiques d'interaction entre le traceur et une région d'intérêt i sont modélisés par un modèle à compartiments.

La Figure 4 représente un exemple d'un modèle à trois compartiments et quatre paramètres pharmacocinétiques.

Le traceur traverse la barrière cellulaire avec une constante de vitesse $K_1$ et peut de la même façon repasser la barrière pour retourner dans le sang avec une constante de vitesse $k_2$. Il est transformé ou se lie à un récepteur dans les cellules avec une constante de vitesse $k_3$. Si cette transformation ou liaison est réversible, la transformation inverse ou dissociation est caractérisée par une constante de vitesse $k_4$.

Les trois compartiments étudiés sont le compartiment plasma-sérum Cp, le compartiment libre $Cf_i$ de la région i et le compartiment lié $Cb_i$ de la région i.

Soit Cp(t), $Cf_i(t)$ et $Cb_i(t)$ les concentrations du traceur dans chacun de ces trois compartiments.

Alors les variations des concentrations du traceur dans les différents compartiments s'écrivent :

$$\frac{dCf_i(t)}{dt} = K_1 Cp(t) - (k_2 + k_3)Cf_i(t) + k_4 Cb_i(t)$$

$$\frac{dCb_i(t)}{dt} = k_3 Cf_i(t) - k_4 Cb_i(t)$$

Soit $Ct_i(t)$ la concentration du traceur dans le tissu de la région i :

$$Ct_i(t) = Cf_i(t) + Cb_i(t)$$

La solution de $Ct_i(t)$ est donnée par :

$$Ct_i(t) = (Cp \otimes h_i)(t)$$

où $h_i(t)$ est la fonction de réponse de la région i au traceur non métabolisé.

Le procédé selon l'invention se propose de prendre en compte la métabolisation éventuelle du traceur, permettant ainsi d'en étendre l'application à un grand nombre de traceurs.

En supposant que le traceur est métabolisé par l'organisme vivant imagé 18 en M métabolites radio-marqués, soit $Ca_m(t)$ la cinétique du métabolite m du traceur dans l'artère, $Cp_m$ la concentration plasmatique du métabolite m du traceur et $h_{i,m}$ la fonction de réponse de la région i au métabolite m du traceur.

Alors le modèle de cinétique $C_i(t)$ dans la région i s'écrit :

$$C_i(t) = Vb_i \times Ca(t) + (1 - Vb_i) \times (Cp \otimes h_i)(t) + \sum_{m=1}^{M} \left( Vb_i \times Ca_m(t) + (1 - Vb_i) \times (Cp_m \otimes h_{i,m})(t) \right)$$

avec $Vb_i$ la fraction de sang dans la région i et $Ca(t)$ la cinétique du traceur non métabolisé dans l'artère.

La troisième étape principale 34 permet d'estimer, pour chaque jeu de cinétiques déterminé à l'étape 30, la fonction d'entrée et les paramètres pharmacocinétiques par résolution du modèle de cinétique défini $C_i(t)$ à partir du jeu de cinétiques déterminé.

Pour cela, le procédé met en oeuvre un algorithme quelconque approprié permettant de minimiser une fonction de coût $d(\{Y_i\}_{1 \leq i \leq I}, \{C_i\}_{1 \leq i \leq I})$ permettant d'évaluer la dissemblance entre les I cinétiques mesurées $\{Y_i\}_{1 \leq i \leq I}$ du jeu de cinétiques déterminé et les I modèles de cinétiques $\{C_i\}_{1 \leq i \leq I}$ correspondants.

La quatrième étape principale 36 permet de déduire la fonction d'entrée finale et les paramètres pharmacocinétiques finaux à partir des fonctions d'entrée estimées et des paramètres pharmacocinétiques estimés à l'étape 34, par exemple en calculant la moyenne ou la médiane des paramètres des fonctions d'entrée estimées et des paramètres pharmacocinétiques estimés.

Le bruit et l'effet de volume partiel qui affectent les cinétiques mesurées génèrent un biais important sur les estimations de la fonction d'entrée et des paramètres pharmacocinétiques. Ce biais augmente généralement lorsque le nombre de paramètres à estimer augmente.

Le procédé selon l'invention offre plusieurs stratégies pour réduire le biais sur les estimations.

Une première stratégie consiste à contraindre le système en introduisant des termes prenant en compte des connaissances a priori sur les paramètres du modèle de cinétique, sur des fonctions de groupes de paramètres, sur la forme de la fonction d'entrée ou artérielle, ou encore sur la cinétique artérielle ou plasmatique des différents métabolites.

Ces termes peuvent comprendre des termes prenant en compte des mesures de métabolites, des termes issus d'une analyse sur une population (par exemple une moyenne et une déviation standard sur les fractions vasculaires $Vb_i$ des régions d'intérêt i), des connaissances a priori sur le traceur (par exemple connaissance du volume de distribution Vd du traceur sur une population donnée), sur l'organisme vivant imagé 18 ou sur les conditions de réalisation de l'examen TEP ou TEMP (par exemple connaissance de la dose injectée DI de traceur), etc.

Ils peuvent être introduits de multiples façons dans le processus d'estimation, en fixant ou en éliminant certains paramètres des équations, ou sous forme de termes supplémentaires dans la fonction de coût d, etc.

Une deuxième stratégie consiste à multiplier les estimations :

- avec le même jeu de cinétiques, c'est-à-dire que l'étape d'estimation 34 est réalisée plusieurs fois pour chaque jeu de cinétiques déterminé à l'étape de détermination 30, comme représenté par la boucle 52 sur la Figure 2. Cette méthode permet de réduire le biais mais de manière très limitée ;
- avec des jeux de cinétiques différents comportant I cinétiques mesurées. En effet, l'étape 30 de détermination d'une pluralité de jeux de cinétiques permet de générer au maximum $C_O^I$ jeux de cinétiques comportant le même nombre I de cinétiques mesurées. Les biais affectant les estimations à partir de ces jeux de cinétiques se compensent ; et
- avec des jeux de cinétiques différents comportant des nombres I différents de cinétiques mesurées. Comme vu précédemment, l'étape 30 de détermination d'une pluralité de jeux de cinétiques permet de générer au maximum

$$\sum_{I=2}^{O} C_O^I \text{ jeux de cinétiques.}$$

Ici encore, les biais affectant les estimations à partir de ces jeux de cinétiques se compensent.

Une troisième stratégie consiste à procéder à une estimation itérative, illustrée par la boucle 54 sur la Figure 2. A la première itération, E fonctions d'entrée et E fonctions de réponse des régions d'intérêt sont estimées de la manière décrite précédemment. Lors des itérations q suivantes, les $E_{q-1}$ fonctions d'entrée et les $E_{q-1}$ fonctions de réponse des régions d'intérêt estimées à l'itération q-1 servent soit à déterminer l'initialisation de chacune des $E_q$ estimations de l'itération q, soit à apporter des contraintes sur l'estimation au moyen de la première stratégie décrite précédemment.

Les étapes 32 de définition du modèle de cinétique et 34 d'estimation de la fonction d'entrée et des paramètres pharmacocinétiques ont été décrites en considérant le modèle de cinétique connu.

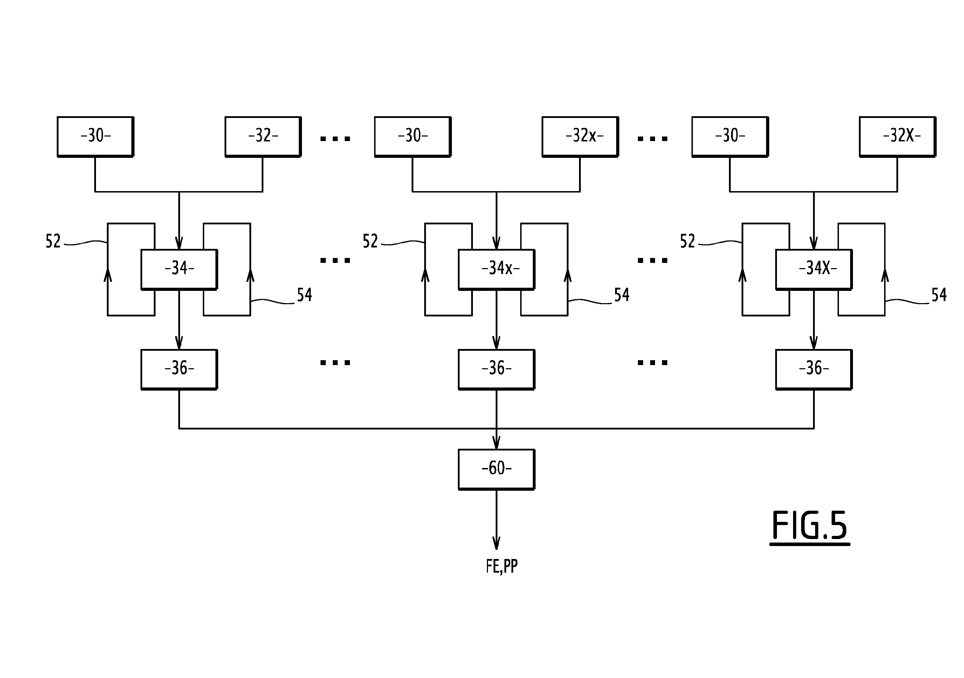

Dans le cas où ce modèle de cinétique n'est pas connu, qu'il s'agisse du comportement pharmacocinétique du traceur non métabolisé et/ou du comportement pharmacocinétique des éventuels métabolites du traceur, le procédé selon l'invention permet d'estimer la fonction d'entrée et les paramètres pharmacocinétiques du traceur en considérant une pluralité de modèles de cinétiques (Figure 5).

Pour cela, le procédé comprend la définition d'une pluralité de modèles de cinétique, des paramètres des modèles de cinétique définis étant formés par les paramètres de la fonction d'entrée et les paramètres pharmacocinétiques, ainsi que la résolution, pour chaque jeu de cinétiques déterminé, des modèles de cinétique définis à partir du jeu de cinétiques déterminé.

Plus précisément, le procédé comprend autant d'étapes supplémentaires 32x de définition d'un modèle de cinétique qu'il y a de modèles de cinétique considérés, x variant ainsi entre 1 et X-1, X étant le nombre total de modèles de cinétique considérés.

Le procédé comprend également autant d'étapes supplémentaires 34x d'estimation de la fonction d'entrée et des paramètres pharmacocinétiques qu'il y a de modèles de cinétique considérés, x variant ainsi entre 1 et X-1 et chaque étape supplémentaire 34x permettant la résolution d'un modèle de cinétique considéré.

Le procédé comprend une étape 60 de choix, parmi la pluralité de modèles de cinétique définis aux étapes 32 et 32x, d'un modèle de cinétique optimal en termes de compromis entre l'adéquation du modèle de cinétique aux données et le faible nombre de paramètres de la fonction d'entrée et des paramètres pharmacocinétiques à extraire.

Cette étape de choix 60 est réalisée par des moyens 62 que comporte le dispositif 10 (Figure 1) et reliés aux moyens de déduction 24.

Un exemple d'application du procédé selon l'invention dans le cadre d'une étude cérébrale chez l'être humain est décrit ci-après.

Une image TEP du cerveau d'un être humain 18 est obtenue par l'appareil d'imagerie 12 et partitionnée en O régions d'intérêt au sein de chacune desquelles la cinétique du traceur est mesurée.

L'étape de détermination 30 est effectuée de la manière décrite précédemment et conduit par exemple à 100 jeux de I cinétiques, avec $2 \leq I \leq O$

Cette étape 30 permet notamment d'éviter de sélectionner un jeu de cinétiques comportant plusieurs cinétiques semblables, par exemple des cinétiques issues de l'hémisphère gauche et de l'hémisphère droit qui sont des ensembles différents mais aux fonctions semblables.

L'étape 32 de définition d'un modèle de cinétique propose un modèle à trois compartiments : un compartiment vasculaire et un compartiment tissulaire comprenant lui-même deux compartiments, l'un où le traceur est libre dans le tissu et l'autre où il est lié de manière spécifique.

On considère ici un traceur ayant un unique métabolite radio-marqué qui ne passe pas la barrière hémato-encéphalique, comme cela est souvent le cas dans les études pharmacocinétiques cérébrales. Le métabolite n'est alors présent que dans le compartiment vasculaire.

On considère également le cas, fréquent, où la concentration plasmatique est égale à la concentration dans l'artère.

On suppose que la fraction vasculaire $Vb_i$ de la région d'intérêt i a une densité de probabilité Gaussienne

connue de moyenne $\mu_{Vbi}$ et de variance $\sigma^2_{Vbi}$, mesurées sur un échantillon de la population à laquelle le sujet imagé 18 appartient.

On suppose également que le volume de distribution Vd du traceur a une densité de probabilité Gaussienne connue de moyenne $u_{vd}$ et de variance $\sigma^2_{Vd}$ .

En incluant le métabolite dans le modèle de cinétique, ce modèle de cinétique dans la région i s'exprime comme :

$$C_i(t) = Vb_i \times \left(Cp(t) + Ca_1(t)\right) + (1 - Vb_i) \times \left(Cp \otimes h_i\right)(t)$$

où $Ca_1$ est la concentration du métabolite dans l'artère.

La fonction de réponse de la région i au traceur non métabolisé s'exprime comme :

$$h_i(t) = a_{i,1}e^{-b_{i,1}t} + a_{i,2}e^{-b_{i,2}t}$$

où $a_{i,1}$, $a_{i,2}$, $b_{i,1}$ et $b_{i,2}$ s'expriment comme des fonctions connues des paramètres pharmacocinétiques $K_1$, $k_2$, $k_3$ et $k_4$.

On choisit pour fonction d'entrée une fonction paramétrique de la forme :

$$Cp(t) = \left(A_1(t - \tau) - A_2 - A_3\right)e^{-\lambda_1(t-\tau)} + A_2 e^{-\lambda_2(t-\tau)} + A_3 e^{-\lambda_3(t-\tau)}$$

où $\tau$ est le délai entre l'injection du traceur et le début de la cinétique plasmatique dans le champ de vue imagé.

La concentration artérielle du traceur métabolisé est donnée par :

$$Ca_1(t) = \begin{cases} t < t_1 : 0 \\ t \ge t_1 : \alpha \times \left(1 - e^{-\beta(t-t_1)}\right) \end{cases}$$

Ici, on considère le modèle comme connu donc il n'y a pas d'étapes supplémentaires 32x à effectuer.

Dans certaines régions, le traceur peut (a) ne pas avoir de récepteur, voire même (b) s'accumuler sans ressortir de la région. Le cas (a) correspond à fixer les paramètres $a_2=b_2=0$, et le cas (b) à fixer $a_2=b_1=b_2=0$.

L'étape 34 d'estimation est ensuite effectuée de la manière suivante.

Soit $\{\Theta_j\}_{1\le j\le J}$ l'ensemble des paramètres déterminant entièrement la forme de la fonction d'entrée, $\{\Phi_{i,k}\}_{1\le k\le K}$ l'ensemble des paramètres de la fonction de réponse de la région i au traceur non métabolisé, $\{\theta_{m,l}\}_{1\le l\le L}$ l'ensemble des paramètres déterminant entièrement la forme de la concentration plasmatique du métabolite m du traceur et $\{\phi_{i,m,n}\}_{1\le n\le N}$ l'ensemble des paramètres de la fonction de réponse de la région i au traceur métabolisé.

On a donc pour la fonction d'entrée :

$$\Theta_1 = \tau, \ \Theta_2 = A_1, \ \Theta_3 = \lambda_1, \ \Theta_4 = A_2, \ \Theta_5 = \lambda_2, \ \Theta_6 = A_3, \ \Theta_7 = \lambda_3$$

Pour les fonctions de réponse des régions i au traceur non métabolisé, on a :

$$\Phi_{i,1} = Vb_i, \ \Phi_{i,2} = a_{i,1}, \ \Phi_{i,3} = a_{i,2}, \ \Phi_{i,4} = b_{i,1}, \ \Phi_{i,5} = b_{i,2}$$

Et pour le modèle du traceur on a :

$$\theta_1 = t_1, \ \theta_2 = \alpha, \ \theta_3 = \beta, \ \{\phi_{i,m,n}\}_{1\le n\le N} = 0$$

La quantité $A_1+A_2$ s'exprime en fonction du volume de distribution Vd du traceur et de la dose injectée DI du traceur :

$$Vd = \frac{DI}{A_1 + A_2}$$

L'estimation des paramètres $\{\Theta_j\}_{1 \leq j \leq 7}$, $\{\Phi_{i,k}\}_{1 \leq k \leq 5}$ et $\{\theta_l\}_{1 \leq l \leq 3}$ à partir d'un jeu $\{\mathbf{Y_i}\}_{1 \leq i \leq I}$ de I cinétiques $Y_i$ déterminé à l'étape 30 est réalisée en minimisant la fonction de coût d :

$$d\left(\{\mathbf{Y_i}\}_{1 \leq i \leq I}, \{\Theta_j\}_{1 \leq j \leq 7}, \{\Phi_{i,k}\}_{1 \leq i \leq I, 1 \leq k \leq 5}, \{\theta_l\}_{1 \leq l \leq 3}\right) = \frac{1}{I}\left(\sum_{i=1}^{I}\left(\frac{w_i}{T}\sum_{t=1}^{T} v_{i,t}\left(Y_i(t) - C_i(t)\right)^2\right) + \delta_i \frac{\left(Vb_i - \mu_{Vb_i}\right)^2}{\sigma_{Vb_i}^2}\right)$$

$$+ \gamma \frac{\left(\frac{DI}{A_1 + A_2} - \mu_{Vd}\right)^2}{\sigma_{Vd}^2}$$

Dans cette fonction de coût d, $w_i$ est le coefficient de recouvrement de la région i, c'est-à-dire la proportion du signal provenant effectivement de la structure i qui contribue à la mesure moyenne de concentration d'activité dans la région i, rendant compte de l'affection de la cinétique $Y_i$ par l'effet de volume partiel, $V_{i,t}$ est la variance de l'erreur commise du fait du bruit statistique dans l'image sur l'estimation de $Y_i(t)$, $\delta_i$ et $\gamma$ sont des poids mesurant l'importance accordée aux a priori respectivement sur les fractions vasculaires des régions et sur le volume de distribution du traceur.
Les paramètres $\{\Theta_j\}_{1 \leq j \leq 7}$, $\{\Phi_{i,k}\}_{1 \leq i \leq I, 1 \leq k \leq 5}$, $\{\theta_l\}_{1 \leq l \leq 3}$ sont estimés par exemple par l'algorithme « Davidon-Fletcher-Powell variable-metric » proposé par le CERN dans sa bibliothèque ROOT sous le nom de « Migrad ».
L'étape 36 de déduction de la fonction d'entrée finale et des paramètres pharmacocinétiques finaux est enfin effectuée. Une fois l'ensemble des E estimations faites, la valeur finale de chaque paramètre $\Theta_j$ de la fonction d'entrée et $\theta_l$ de la concentration artérielle du métabolite est choisie comme la médiane des E valeurs estimées pour ce paramètre.
Les autres paramètres $\Phi_{i,k}$ sont ré-estimés comme décrit pour l'étape d'estimation 34, mais en fixant les paramètres $\Theta_j$ de la fonction d'entrée et $\theta_l$, de la concentration artérielle auxdites valeurs finales estimées, permettant une meilleure estimation des paramètres pharmacocinétiques.
En ce qui concerne la réduction du biais sur les paramètres estimés, le système est ici contraint par le choix d'une forme analytique paramétrique pour la fonction d'entrée, mais également pour la cinétique du métabolite dans l'artère.
Les connaissances a priori sur les fractions vasculaires $Vb_i$ des régions i et sur le volume de distribution Vd du traceur introduites dans la fonction de coût d sous la forme des termes ; $\delta_i \dfrac{\left(Vb_i - \mu_{Vb_i}\right)^2}{\sigma_{Vb_i}^2}$ et $\gamma \dfrac{\left(\frac{DI}{A_1 + A_2} - \mu_{Vd}\right)^2}{\sigma_{Vd}^2}$ contraignent la minimisation.
S'il est connu que la concentration du traceur s'équilibre entre la veine et l'artère, on peut ajouter à la fonction de coût d le terme $\varsigma \times \sum_{l=1}^{L} \dfrac{\left((Cp(t_l) + Ca_1(t_l)) - Cv(t_l)\right)^2}{2 \times \sigma_v^2}$ , où Cv est la concentration veineuse du traceur mesurée à partir du moment où l'équilibre veine-artère est réalisé et mesurée en L points de temps $t_l$ différents, $\sigma_v^2$ est l'incertitude sur les prélèvements veineux due à la l'imprécision statistique du compteur gamma et $\zeta$ est le poids mesurant l'importance accordée aux prélèvements veineux.
Les prélèvements veineux sont à différencier des prélèvements artériels, ces derniers étant beaucoup plus difficiles et inconfortables à réaliser pour le sujet imagé 18.
Si l'on est en mesure d'estimer la cinétique artérielle $Ca_{meas}$ du traceur (métabolisé et non-métabolisé) directement à partir de l'image et d'une manière fiable, alors la quantité $Cp(t) + Ca_1(t)$ peut être remplacée par $Ca_{meas}$ dans l'expression du modèle de cinétique $C_i(t)$, ce qui permet de diminuer de trois le nombre de paramètres à estimer. Une fois les autres paramètres estimés, ces trois paramètres liés à la concentration artérielle du métabolite pourront être estimés en fixant tous les autres paramètres.

L'étape d'estimation 34 est réalisée plusieurs fois pour chaque jeu de cinétiques, par exemple cinq fois, afin de trouver les différentes solutions du modèle de cinétique défini qui correspondent aux différents minimas de la fonction de coût d lorsque celle-ci en présente plusieurs.

Les jeux de cinétiques déterminés sont tous différents les uns des autres, certains jeux de cinétiques comportant le même nombre I de cinétiques mesurées et d'autres jeux de cinétiques comportant un nombre I différent de cinétiques mesurées.

Par exemple ici, le nombre I de cinétiques mesurées par jeu de cinétiques varie entre 3 et O, ce qui correspond à un nombre total maximal de $\sum_{l=3}^{O} C_O^l$ jeux de cinétiques, ce qui donne 968 jeux de cinétiques pour O=10 et 32647 jeux de cinétiques pour O=15.

Un nombre aussi important d'estimations n'est pas nécessaire, car le gain en précision en ajoutant un jeu de cinétiques supplémentaire diminue avec le nombre de jeux de cinétiques déjà utilisés. Par exemple, le nombre de jeux de cinétiques peut être limité à au maximum 100.

Concernant l'estimation itérative, les paramètres à extraire sont ici estimés en une unique itération.

Lorsque le modèle de cinétique n'est pas connu, des modèles de cinétique simplifiés $\chi$ sont testés au moyen des étapes 32x et 34x.

L'étape 60 de choix d'un modèle de cinétique optimal permet ensuite de choisir, parmi tous les modèles $\chi$ testés, le modèle le plus simple qui rend le mieux compte des processus physiologiques sous-jacents aux cinétiques mesurées.

Par exemple, le modèle de cinétique choisi pour chaque région i est celui vérifiant :

$$\text{K} = \arg\max_{\chi} \left( d\left( \{\mathbf{Y}_i\}_{1 \leq i \leq I}, \{\Theta_j^{(\chi)}\}_{1 \leq j \leq J}, \{\Phi_{i,k}^{(\chi)}\}_{1 \leq i \leq I, 1 \leq k \leq K}, \{\theta_l^{(\chi)}\}_{1 \leq l \leq L} \right) \times \left( J + L + \frac{I \times K}{2} \right) \right) \text{ où}$$

$\{\Theta_j^{(\chi)}\}_{1 \leq j \leq J}, \{\Phi_{i,k}^{(\chi)}\}_{1 \leq i \leq I, 1 \leq k \leq K}, \{\theta_l^{(\chi)}\}_{1 \leq l \leq L}$ sont les paramètres finaux déduits à l'étape 36 pour le modèle $\chi$, pour la fonction d'entrée et pour la région i.

Le procédé selon l'invention a été testé sur des données simulées ainsi que sur des données expérimentales.

Les données simulées sont constituées de N cinétiques générées de manière aléatoire selon un modèle à trois compartiments et la fonction d'entrée choisie est une fonction d'entrée paramétrique telle que décrite précédemment.

Les fractions vasculaires Vb_i des régions i sont tirées aléatoirement de manière à rester plausible en fonction du sujet imagé 18. Pour les études cérébrales, les fractions vasculaires sont de 1% (tissu conjonctif), 2% (matière blanche), 3% (ganglions de la base) et 4% (cortex), alors que pour les études corps entier chez le rongeur, les fractions vasculaires varient entre 5% (muscle) et 10% (foie, poumons), voire 15% (reins) ou 40% (coeur).

Les paramètres fixés du procédé (on rappelle qu'il est mathématiquement impossible d'estimer la fonction d'entrée et/ou les paramètres pharmacocinétiques sans fixer certains paramètres du modèle de cinétique) sont les fractions vasculaires Vb_i des régions i, supposées connues pour le cerveau humain et estimées sur une population pour le rongeur, ainsi que le temps de délai $\tau$ entre l'injection du traceur et la disponibilité du traceur aux régions i. Ce temps $\tau$ est très faible chez le rongeur, mais plus long chez l'homme.

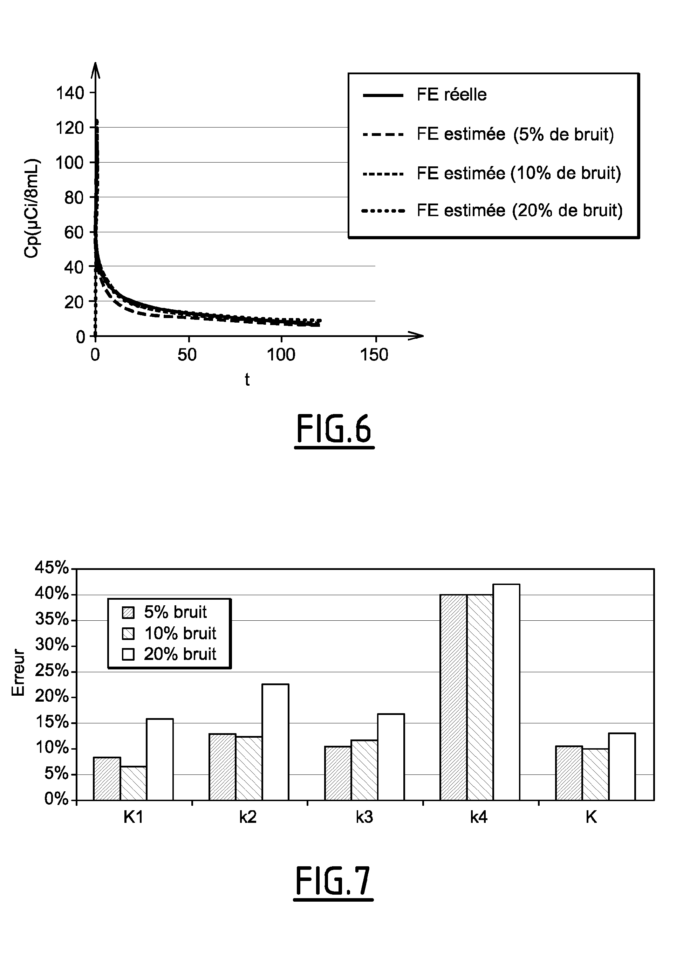

La Figure 6, qui représente les résultats obtenus pour la fonction d'entrée pour différentes valeurs de bruit sur des données simulées type cerveau humain et compare ces résultats à la fonction d'entrée réelle, indique que la fonction d'entrée est bien estimée quelle que soit l'importance du bruit qui affecte les cinétiques d'entrée, et ce sans recourir à des prélèvement veineux et/ou artériels.

Les paramètres pharmacocinétiques K1, k2, k3 et k4 sont tirés aléatoirement dans un intervalle cohérent avec des valeurs connues. Pour les données cérébrales, k4 a été choisi petit pour simuler des cinétiques d'examen au traceur FDG.

La Figure 7 représente l'erreur relative moyenne sur chacun des paramètres pharmacocinétiques en fonction du niveau de bruit avec des données simulées type cerveau humain.

L'erreur relative importante obtenue sur k4 est due à la très faible valeur de ce paramètre, une faible erreur absolue donnant une forte erreur relative.

Le macro-paramètre K est un paramètre permettant de mesurer la concentration régionale de glucose :

métabolisme régional du glucose (CMRglu) = $\dfrac{K_1 k_3}{k_2 + k_3} Cg_p/LC = KxCg_p/LC$

où Cg_p est la concentration plasmatique de glucose, aisée à obtenir par contrôle de la glycémie durant l'examen, et LC est la constante localisée (ou « lumped constant » en anglais) qui permet de passer du K du modèle du FDG au K du modèle du glucose.

La grandeur $K = \dfrac{K_1 k_3}{k_2 + k_3}$ est donc la principale grandeur d'intérêt. Une erreur de 10% sur K est raisonnable compte tenu du fait que l'on ne recourt pas à des prélèvements veineux et/ou artériels.

Le procédé a également été testé sur des données expérimentales type cerveau humain et avec le traceur FDG et comparé à des prélèvements artériels.

Les fractions vasculaires Vb$_i$ des régions i sont ici fixées à des valeurs supposées connues, mais devront dans les faits être estimées plus précisément pour améliorer les résultats. Le délai τ a été fixé au milieu de l'entrée du signal TEP dans le cerveau. Une méthode d'estimation plus fine de tau doit également être mise en place afin d'améliorer les résultats.

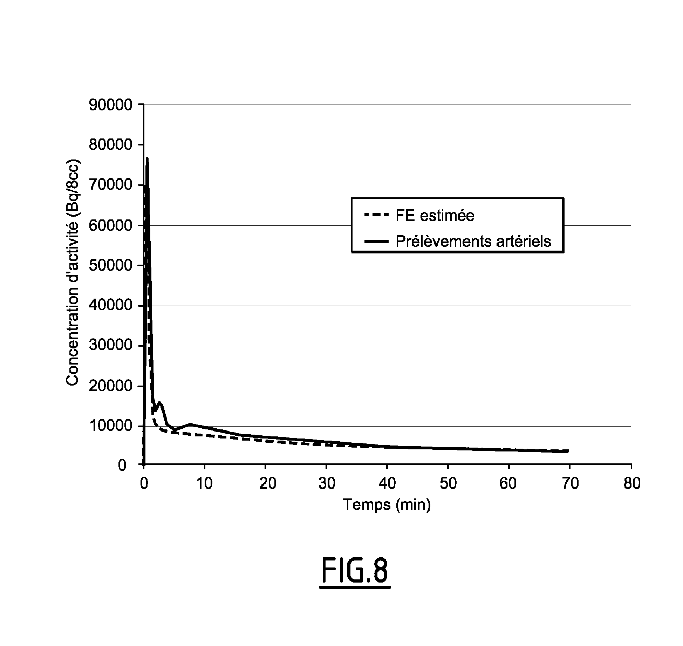

La Figure 8, qui représente les résultats obtenus pour la fonction d'entrée sur des données expérimentales type cerveau humain et compare ces résultats à la fonction d'entrée réelle obtenue par des prélèvements artériels, qui constituent la référence, indique que la fonction d'entrée est bien estimée, avec une légère sous-estimation dans sa partie centrale, en particulier au moment du second pic de la fonction d'entrée correspondant au « lavage de la seringue », pic qui peut être évité si l'on n'a pas besoin de connaître précisément l'activité injectée, le « lavage de la seringue » consistant à ajouter du sérum physiologique dans la seringue et à l'injecter pour pousser le traceur résiduel resté dans les cathéters.

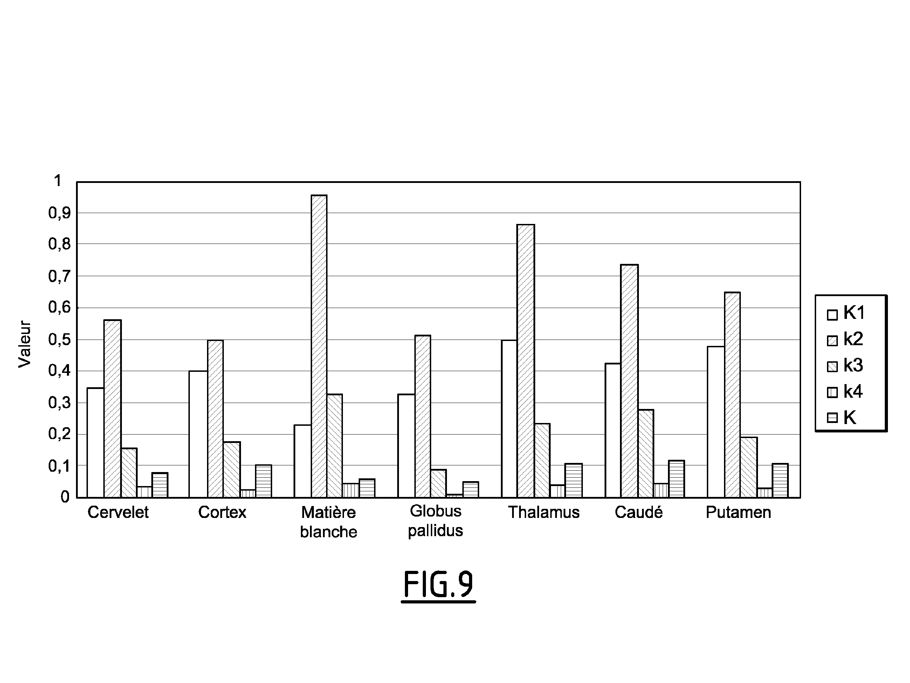

La Figure 9 représente les résultats obtenus pour chacun des paramètres pharmacocinétiques ainsi que pour le macro-paramètre K avec des données expérimentales type cerveau humain.

On ne dispose pas ici de référence, mais les paramètres peuvent être comparés entre régions. Il paraît raisonnable que le facteur K soit très proche pour le cortex, le thalamus, le caudé et le putamen, et plus bas pour la matière blanche et le globus pallidus, avec une valeur intermédiaire pour le cervelet.

D'autre part, le fait que K1 soit plus faible pour la matière blanche que pour le cortex paraît également plausible.

L'invention propose donc un procédé à caractère invasif minimal qui permet d'extraire directement à partir d'une image de mesure de la cinétique d'un principe actif et avec le moins de biais possible la fonction d'entrée simultanément avec les paramètres pharmacocinétiques de ce principe actif.

En minimisant une distance entre plusieurs cinétiques mesurées et les cinétiques obtenues comme solution d'un modèle à compartiments, en multipliant les estimations à partir d'un nombre important de jeux de cinétiques différents et en introduisant des a priori dans l'estimation autres que des prélèvements artériels, le procédé selon l'invention permet de réduire les erreurs commises lors d'une estimation et d'améliorer ainsi la robustesse de l'estimation.

De plus, la prise en compte du comportement pharmacocinétique des éventuels métabolites du traceur permet d'appliquer ce procédé à la quasi-totalité des traceurs existants et à venir, tant dans des études corps entier qu'en intracérébral ou d'autres organes.

Par ailleurs, le procédé selon l'invention permet de modifier la nature même de l'information apportée par les examens TEP ou TEMP et qui est d'intérêt pour le praticien (médecin, pharmacologue, biologiste, etc.), qui ne donnera plus uniquement des mesures de radioactivité, mais directement les paramètres du modèle qui sous-tendent ces mesures de radioactivité.

Plusieurs applications médicales peuvent être envisagées : la pharmacologie, le développement de médicaments, le diagnostic médical, la réduction du nombre d'examens, etc.

La pharmacologie est une branche de la recherche médicale dont le but est la compréhension de l'effet du médicament sur l'organisme (pharmacodynamique) et de l'effet de l'organisme sur le médicament (pharmacocinétique). Elle permet ainsi de caractériser des pathologies, des médicaments et des traceurs. L'obtention de paramètres pharmacocinétiques fins est une étape essentielle de la pharmacocinétique. L'enjeu du présent procédé pour la pharmacocinétique est crucial, car les prélèvements artériels sont de moins en moins pratiqués pour des raisons de sécurité et de confort du sujet imagé.

Environ 39% des médicaments n'atteignent pas le marché pour des raisons de mauvaise pharmacocinétique. L'obtention de paramètres pharmacocinétiques dès la phase préclinique et à chaque étape de la phase clinique pourrait permettre, avec une grande richesse d'information et des mesures physiologiquement quantitatives, d'écarter ou de modifier aussitôt que possible les molécules candidates. Cet élément est d'autant plus crucial pour le développement des médicaments que les coûts de développement restent actuellement les mêmes alors que de moins en moins de médicaments arrivent sur le marché, du fait des demandes croissantes de preuves scientifiques et de sûreté de la part des organismes officiels.

La possibilité d'obtenir des paramètres pharmacocinétiques sans recourir à des prélèvements artériels ouvre à la pharmacocinétique les portes du diagnostic clinique. Les conséquences, en termes d'indices, de sensibilité et de spécificité diagnostiques, sont susceptibles de révolutionner l'usage de la TEP.

La directive 86/609/EEC sur la protection des animaux utilisés pour des buts scientifiques expérimentaux et autres demandes à ce que les expérimentations animales soient aussi réduites que possible. Que ce soit chez l'homme ou chez l'animal, le coût d'un examen TEP ou TEMP est de plus extrêmement élevé, et la réduction du nombre d'examens s'accompagnerait d'une réduction substantielle des coûts pour la recherche médicale. Or, il est connu que, pour la mesure d'un même effet d'un médicament avec le même degré de certitude, l'estimation de paramètres pharmacocinétiques permet de réduire d'environ un ordre de grandeur le nombre d'examens nécessaires comparée à une simple mesure de SUV (Standard Uptake Value).

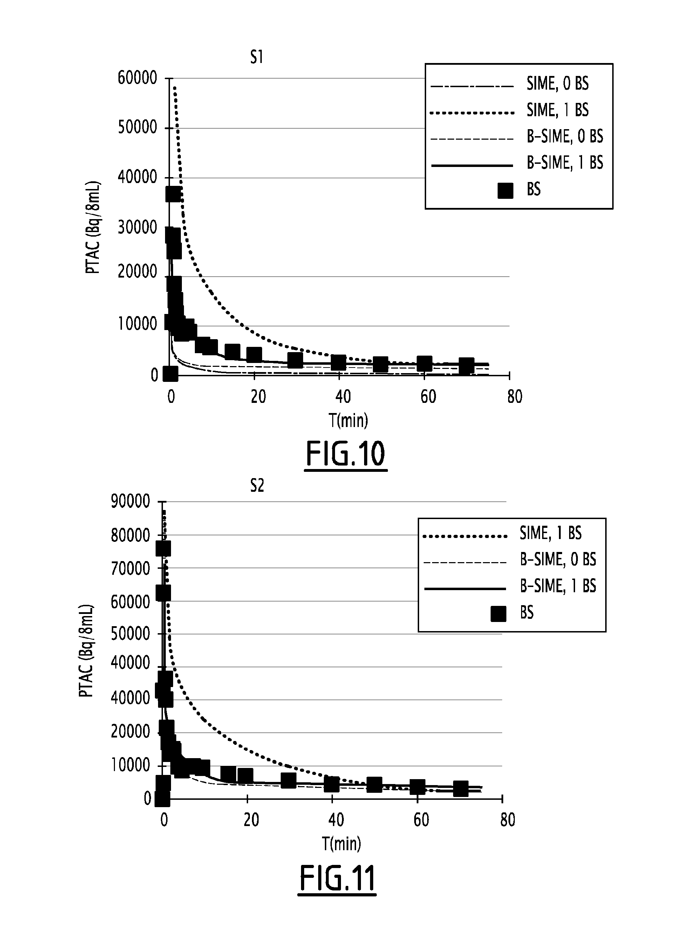

La figure 10 représente l'évolution de l'activité dans le plasma artériel (« PTAC » pour « Plasma Time Activity Curve ») exprimée en Bq/ML, d'un sujet sain nommé S1, au cours du temps T, exprimé en minutes, selon différentes méthodes dont une méthode dans un mode de mise en oeuvre de l'invention.

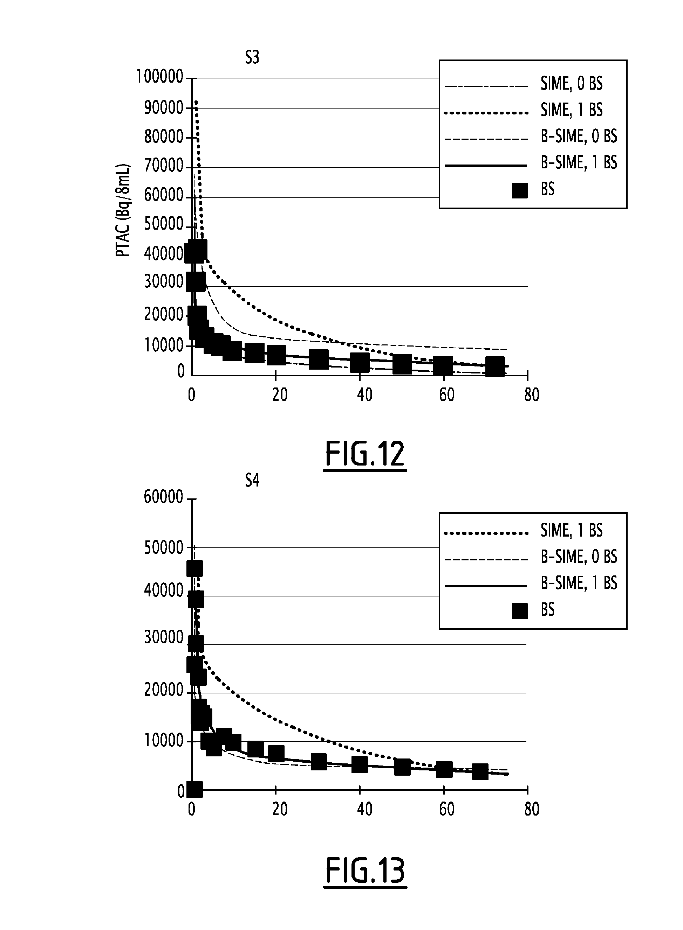

Similairement, les figures 11 à 13 représentent l'évolution de l'activité dans le plasma artériel de sujets sains respectifs S2, S3 et S4 au cours du temps, selon différentes méthodes dont une méthode dans un mode de mise en oeuvre de l'invention.

La méthode dans un mode de mise en oeuvre de l'invention, nommée B-SIME (Bootstrap approach for the SIME method) sur les figures 10 à 13, a été validée sur 4 sujets sains ayant eu un examen TEP (Tomographie d'Emission de Positons), un examen IRM (Imagerie par Résonance magnétique Nucléaire) et des prélèvements au poignet de sang artériel durant l'acquisition (BS pour Blood Sample).

Les méthodes SIME et B-SIME ont été comparées sur les quatre sujets sains S1, S2, S3, S4 ayant passé un examen IRM T1 sur l'imageur GE Signa 1.5T et un examen TEP d'une heure et 10 minutes avec un découpage temporel de 1 x 20 s, 10 x 10 s, 2 x 30 s, 2 x 2 min 30 s, 4 x 5 min, puis 1 x 5 min à 50 min et 1 x 5 min à 1 h 5 min. Les images ont été reconstruites avec la méthode OP-OSEM avec des voxels de taille 2.45 x 2.45 x 2.45 mm3. Les images IRM ont été segmentées automatiquement en 15 structures cérébrales et corrigé du volume partiel au moyen de la méthode Geometric Transfer Matrix (V. Frouin, JNM, 2002).

Les prélèvements sanguins ont été pris à intervalles irréguliers (donnés ici en minutes après l'injection du traceur.Les méthodes SIME et B-SIME ont été comparées sur quatre sujets sains ayant passé un examen IRM T1 sur l'imageur GE Signa 1.5T et un examen TEP d'une heure et 10 minutes avec un découpage temporel de 1 x 20 s, 10 x 10 s, 2 x 30 s, 2 x 2 min 30 s, 4 x 5 min, puis 1 x 5 min à 50 min et 1 x 5 min à 1 h 5 min. Les images ont été reconstruites avec la méthode OP-OSEM avec des voxels de taille 2.45 x 2.45 x 2.45 mm3. Les images IRM ont été segmentées automatiquement en 15 structures cérébrales et corrigé du volume partiel au moyen de la méthode Geometric Transfer Matrix (V. Frouin, JNM, 2002).

Les prélèvements sanguins ont été pris à intervalles irréguliers (donnés ici en minutes après l'injection du traceur)

Temps (minute après injection)

| S1 | S2 | S3 | S4 |
|------|------|------|-------|
| 0,15 | 0,07 | 0,10 | 0,10 |
| 0,35 | 0,20 | 0,30 | 0,25 |
| 0,47 | 0,30 | 0,40 | 0,35 |
| 0,62 | 0,42 | 0,58 | 0,48 |
| 0,78 | 0,52 | 0,83 | 0,60 |
| 0,97 | 0,63 | 1,07 | 0,72 |
| 1,12 | 0,73 | 1,37 | 1,07 |
| 1,28 | 1,00 | 1,53 | 1,18 |
| 1,43 | 1,12 | 1,68 | 1,32 |
| 1,58 | 1,40 | 1,87 | 1,63 |
| 1,82 | 1,67 | 2,03 | 1,77 |
| 2,01 | 2,08 | 2,23 | 2,02 |
| 2,70 | 2,62 | 2,52 | 2,52 |
| 3,01 | 3,02 | 3,10 | 3,00 |
| 3,97 | 3,87 | 4,33 | 4,00 |
| 4,80 | 5,07 | 5,20 | 5,42 |
| 7,70 | 7,57 | 7,38 | 7,53 |
| 9,53 | 9,58 | 9,78 | 10,00 |

(suite)

| S1 | S2 | S3 | S4 |
|---|---|---|---|
| 14,87 | 15,70 | 14,88 | 15,17 |
| 19,97 | 19,78 | 19,52 | 20,17 |
| 29,75 | 29,93 | 29,80 | 30,13 |
| 39,78 | 39,97 | 39,85 | 40,00 |
| 49,68 | 49,97 | 50,00 | 50,07 |
| 60,10 | 60,03 | 59,83 | 60,02 |
| 70,10 | 70,18 | 72,33 | 68,73 |

Il a été réalisé au total 4 itérations avec pour chaque itération 1050 estimations : 300 avec 3 organes par jeu de cinétiques (OPJ), 250 avec 4 OPJ, 200 avec 5 OPJ, 150 avec 6 OPJ, 100 avec 7 OPJ et 50 avec 8 OPJ, soit au total 4200 estimations. A la fin de chaque estimation, les paramètres de la fonction d'entrée finale ont été choisis égaux à la médiane des mêmes paramètres des fonction d'entrée estimées pondérées par la valeur prise au minimum par la fonction à minimiser. Le paramètre ayant la plus faible variation relative (TroisièmeQuartile - PremierQuartile)/Médiane à la fin d'une itération est fixé pour l'itération suivante.

La méthode selon l'invention a permis de retrouver la cinétique artérielle avec une bonne précision chez chacun des quatre sujets en utilisant un unique prélèvement veineux lors de l'estimation, contrairement à la méthode originelle SIME (SIMultaneous Estimation of the input function and of the pharmacological parameters) sur les figures 10 à 13), qui surestime systématiquement la cinétique artérielle avec également un prélèvement veineux.

## Revendications

1. Procédé d'extraction simultanée de la fonction d'entrée (FE) et des paramètres pharmacocinétiques (PP) d'un principe actif dans une structure d'un organisme vivant (18) à partir d'une image de mesure de la cinétique du principe actif dans la structure obtenue par un appareil d'imagerie (12), l'image comprenant une pluralité de régions d'intérêt à l'intérieur de chacune desquelles la cinétique du principe actif est mesurée, **caractérisé en ce qu'**il comprend les étapes suivantes :

   - détermination (30) d'une pluralité de jeux de cinétiques à partir des cinétiques mesurées, les jeux de cinétiques déterminés étant différents les uns des autres et chaque jeu de cinétiques déterminé comportant plusieurs cinétiques mesurées ;
   - définition (32) d'au moins un modèle de cinétique, des paramètres du modèle de cinétique défini étant formés par les paramètres de la fonction d'entrée et les paramètres pharmacocinétiques ;
   - estimation (34), pour chaque jeu de cinétiques déterminé, de la fonction d'entrée et des paramètres pharmacocinétiques par résolution du modèle de cinétique défini à partir du jeu de cinétiques déterminé ; et
   - déduction (36) de la fonction d'entrée finale et des paramètres pharmacocinétiques finaux à partir des fonctions d'entrée estimées et des paramètres pharmacocinétiques estimés.

2. Procédé selon la revendication 1, **caractérisé en ce que** l'étape de détermination (30) de la pluralité de jeux de cinétiques comprend les étapes suivantes :

   - constitution (40) de tous les jeux de cinétiques possibles à partir des cinétiques mesurées ; et
   - sélection (42), parmi les jeux de cinétiques constitués, d'un nombre prédéterminé de jeux de cinétiques, cette étape comprenant les étapes suivantes :

     • évaluation (44), pour chaque jeu de cinétiques constitué, d'un critère de sélection de jeux de cinétiques permettant d'évaluer leur risque d'introduire des erreurs dans l'estimation de la fonction d'entrée et des paramètres pharmacocinétiques ;
     • classement (46) des jeux de cinétiques constitués selon une règle du critère de sélection maximal ; et
     • choix (48) du nombre prédéterminé des premiers jeux de cinétiques classés.

3. Procédé selon la revendication 2, **caractérisé en ce que** le critère de sélection de jeux de cinétiques permet de sélectionner les jeux de cinétiques dont les cinétiques mesurées sont différentes les unes des autres en termes de forme et/ou d'amplitude, et/ou dont les cinétiques mesurées sont peu affectées par l'effet de volume partiel et/ou dont les cinétiques mesurées présentent un fort rapport signal sur bruit.

4. Procédé selon l'une quelconque des revendications 1 à 3, **caractérisé en ce que** le modèle de cinétique défini prend en compte la métabolisation du principe actif dans la structure de l'organisme vivant imagé (18).

5. Procédé selon l'une quelconque des revendications 1 à 4, **caractérisé en ce que** l'étape d'estimation de la fonction d'entrée et des paramètres pharmacocinétiques est réalisée plusieurs fois pour chaque jeu de cinétiques déterminé de manière à trouver les différentes solutions du modèle de cinétique défini.

6. Procédé selon l'une quelconque des revendications 1 à 5, **caractérisé en ce que** les jeux de cinétiques déterminés comprennent des jeux de cinétiques comportant le même nombre de cinétiques mesurées.

7. Procédé selon l'une quelconque des revendications 1 à 6, **caractérisé en ce que** les jeux de cinétiques déterminés comprennent des jeux de cinétiques comportant un nombre différent de cinétiques mesurées.

8. Procédé selon l'une quelconque des revendications 1 à 7, **caractérisé en ce que** l'étape d'estimation (34) de la fonction d'entrée et des paramètres pharmacocinétiques comprend la minimisation d'une fonction de coût (d) permettant d'évaluer la dissemblance entre le jeu de cinétiques déterminé et le modèle de cinétique défini.

9. Procédé selon l'une quelconque des revendications 1 à 8, **caractérisé en ce que** l'étape d'estimation (34) de la fonction d'entrée et des paramètres pharmacocinétiques est réalisée suivant une boucle itérative (54) dans laquelle les fonctions d'entrée estimées et les paramètres pharmacocinétiques estimés lors d'une itération permettent de déterminer l'initialisation des fonctions d'entrée estimées et des paramètres pharmacocinétiques estimés à l'itération suivante et/ou d'apporter des contraintes sur les fonctions d'entrée estimées et les paramètres pharmacocinétiques estimés à l'itération suivante.

10. Procédé selon l'une quelconques des revendications 1 à 9, **caractérisé en ce que** :

    - l'étape de définition (32) d'au moins un modèle de cinétique comprend la définition (32x) d'une pluralité de modèles de cinétique, des paramètres des modèles de cinétique définis étant formés par les paramètres de la fonction d'entrée et les paramètres pharmacocinétiques ;
    - l'étape d'estimation (34) de la fonction d'entrée et des paramètres pharmacocinétiques comprend, pour chaque jeu de cinétiques déterminé, la résolution (34x) des modèles de cinétique définis à partir du jeu de cinétiques déterminé,
    et le procédé comprend une étape de choix (60), parmi la pluralité de modèles de cinétique, d'un modèle de cinétique optimal en termes de compromis entre l'adéquation du modèle de cinétique aux données et le faible nombre de paramètres de la fonction d'entrée et des paramètres pharmacocinétiques à extraire.

11. Procédé selon l'une quelconque des revendications 1 à 10, **caractérisé en ce que** l'étape de définition (32) d'au moins un modèle de cinétique comprend, outre le choix de la forme du modèle de cinétique, l'introduction dans le modèle de cinétique de contraintes résultant de connaissances a priori non liées à des prélèvements artériels effectués sur l'organisme vivant imagé (18), les contraintes permettant de fixer au moins un paramètre du modèle de cinétique tel que les fractions vasculaires ($Vb_i$) des régions d'intérêt.

12. Procédé selon l'une quelconque des revendications 1 à 11, **caractérisé en ce que** l'étape d'estimation (34) de la fonction d'entrée et des paramètres pharmacocinétiques comprend l'introduction dans la résolution du modèle de cinétique de contraintes résultant de connaissances a priori non liées à des prélèvements artériels effectués sur l'organisme vivant imagé (18), les contraintes permettant d'ajouter des termes connus dans la résolution du modèle de cinétique tels que des termes prenant en compte des connaissances a priori sur les fractions vasculaires ($Vb_i$) des régions d'intérêt et/ou sur le volume de distribution (Vd) du principe actif sur une population donnée.

13. Procédé selon l'une quelconque des revendications 1 à 12, **caractérisé en ce que** l'image de mesure de la cinétique du principe actif dans la structure est obtenue à partir d'une séquence temporelle d'images tridimensionnelles obtenues par tomographie par émission de positons (TEP) ou par tomographie par émission monophotonique (TEMP).

14. Support de stockage d'information (26) comprenant un code (28) pour extraire simultanément la fonction d'entrée (FE) et les paramètres pharmacocinétiques (PP) d'un principe actif dans une structure d'un organisme vivant (18) à partir d'une image de mesure de la cinétique du principe actif dans la structure obtenue par un appareil d'imagerie (12), l'image comprenant une pluralité de régions d'intérêt à l'intérieur de chacune desquelles la cinétique du principe actif est mesurée, **caractérisé en ce que** le code (28) comprend des instructions pour :

- déterminer une pluralité de jeux de cinétiques à partir des cinétiques mesurées, les jeux de cinétiques déterminés étant différents les uns des autres et chaque jeu de cinétiques déterminé comportant plusieurs cinétiques mesurées ;
- définir au moins un modèle de cinétique, des paramètres du modèle de cinétique défini étant formés par les paramètres de la fonction d'entrée et les paramètres pharmacocinétiques ;
- estimer, pour chaque jeu de cinétiques déterminé, la fonction d'entrée et les paramètres pharmacocinétiques par résolution du modèle de cinétique défini à partir du jeu de cinétiques déterminé ; et
- déduire la fonction d'entrée finale et les paramètres pharmacocinétiques finaux à partir des fonctions d'entrée estimées et des paramètres pharmacocinétiques estimés.

15. Dispositif (10) destiné à extraire simultanément la fonction d'entrée (FE) et les paramètres pharmacocinétiques (PP) d'un principe actif dans une structure d'un organisme vivant (18) à partir d'une image de mesure de la cinétique du principe actif dans la structure obtenue par un appareil d'imagerie (12), l'image comprenant une pluralité de régions d'intérêt à l'intérieur de chacune desquelles la cinétique du principe actif est mesurée, **caractérisé en ce qu'**il comprend :

- un appareil d'imagerie (12) ; et
- un système de traitement de données (14) relié à l'appareil d'imagerie (12) et comprenant :

• des moyens (16) pour déterminer une pluralité de jeux de cinétiques à partir des cinétiques mesurées, les jeux de cinétiques déterminés étant différents les uns des autres et chaque jeu de cinétiques déterminé comportant plusieurs cinétiques mesurées ;
• des moyens (20) pour définir au moins un modèle de cinétique, des paramètres du modèle de cinétique défini étant formés par les paramètres de la fonction d'entrée et les paramètres pharmacocinétiques ;
• des moyens (22) pour estimer, pour chaque jeu de cinétiques déterminé, la fonction d'entrée et les paramètres pharmacocinétiques par résolution du modèle de cinétique défini à partir du jeu de cinétiques déterminé ; et
• des moyens (24) pour déduire la fonction d'entrée finale et les paramètres pharmacocinétiques finaux à partir des fonctions d'entrée estimées et des paramètres pharmacocinétiques estimés.

FE,PP

## FIG.1

FE,PP

FIG.2

FIG.3

FIG.4

FIG.5

**FIG.6**

**FIG.7**

FIG.8

FIG.9

FIG.10

FIG.11

FIG.12

FIG.13

**EP 2 365 455 A1**

| | |
|---|---|
| Europäisches Patentamt / European Patent Office / Office européen des brevets | **RAPPORT DE RECHERCHE EUROPEENNE** | Numéro de la demande / EP 11 30 5247 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | DAGAN FENG ET AL: "A Technique for Extracting Physiological Parameters and the Required Input Function Simultaneously from PET Image Measurements: Theory and Simulation Study", IEEE TRANSACTIONS ON INFORMATION TECHNOLOGY IN BIOMEDICINE, IEEE SERVICE CENTER, LOS ALAMITOS, CA, US, vol. 1, no. 4, 1 décembre 1997 (1997-12-01), pages 243-254, XP011028290, ISSN: 1089-7771 * le document en entier * ----- | 1-15 | INV. G06F19/00 A61B5/0275 G06K9/46 G06T7/00 |
| A | WO 2009/019535 A1 (KONINKL PHILIPS ELECTRONICS NV [NL]; PHILIPS INTELLECTUAL PROPERTY [DE] 12 février 2009 (2009-02-12) * page 1, ligne 7 - page 2, ligne 15 * * page 4, ligne 23 - page 10, ligne 7 * ----- | 1-15 | |
| A | WO 2007/132242 A1 (STIFTELSEN UNIVERSITETSFORSKNI [NO]; GODDARD CHRISTOPHER [GB]; TAXT TO) 22 novembre 2007 (2007-11-22) * page 2, alinéa 2 - page 14, alinéa 5 * ----- | 1-15 | DOMAINES TECHNIQUES RECHERCHES (IPC) G06F A61B G06K G06T |
| A | MAROY R ET AL: "Segmentation of Rodent Whole-Body Dynamic PET Images: An Unsupervised Method Based on Voxel Dynamics", IEEE TRANSACTIONS ON MEDICAL IMAGING, IEEE SERVICE CENTER, PISCATAWAY, NJ, US, vol. 27, no. 3, 1 mars 2008 (2008-03-01), pages 342-354, XP011202052, ISSN: 0278-0062 * le document en entier * ----- -/-- | 1-15 | |

Le présent rapport a été établi pour toutes les revendications

| Lieu de la recherche | Date d'achèvement de la recherche | Examinateur |
|---|---|---|
| La Haye | 8 avril 2011 | Türkeli, Yasemin |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 11 30 5247

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | ANDREW WEBB ED - WEBB A: "Statistical pattern recognition, Feature selection", 31 décembre 1999 (1999-12-31), STATISTICAL PATTERN RECOGNTION, LONDON [U.A.] : ARNOLD, PAGE(S) 215226, XP002554368, ISBN: 978-0-340-74164-1 pages 215-226, * page 215 - page 217, 8.2 "Feature selection"; 8.2.1 "Feature selection criteria" * ----- | 2 | |
| | | | **DOMAINES TECHNIQUES RECHERCHES (IPC)** |

Le présent rapport a été établi pour toutes les revendications

| Lieu de la recherche | Date d'achèvement de la recherche | Examinateur |
|---|---|---|
| La Haye | 8 avril 2011 | Türkeli, Yasemin |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 11 30 5247

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-04-2011

| Document brevet cité au rapport de recherche | | Date de publication | Membre(s) de la famille de brevet(s) | | Date de publication |
|---|---|---|---|---|---|
| WO 2009019535 | A1 | 12-02-2009 | CN<br>EP<br>JP | 101772783 A<br>2174292 A1<br>2010536017 T | 07-07-2010<br>14-04-2010<br>25-11-2010 |
| WO 2007132242 | A1 | 22-11-2007 | CN<br>EP<br>JP<br>US | 101506842 A<br>2038841 A1<br>2009537201 T<br>2009297008 A1 | 12-08-2009<br>25-03-2009<br>29-10-2009<br>03-12-2009 |

EPO FORM P0460

Pour tout renseignement concernant cette annexe : voir Journal Officiel de l'Office européen des brevets, No.12/82